# EUROPÄISCHE PATENTSCHRIFT

(11) **EP 1 342 713 B1**
(45) Veröffentlichungstag und Bekanntmachung des Hinweises auf die Patenterteilung: **04.01.2012**
(21) Anmeldenummer: 03003234.6
(22) Anmeldetag: 21.02.2003
(51) Int. Cl.: C07C 205/06, C07C 209/36, C07C 263/10

(54) **Verfahren zur Herstellung eines Dinitronaphthalin-Isomerengemisches mit hohem Anteil an 1,5-Dinitronaphtalin**
Method for producing a dinitronaphthalene-isomer mixture having an increased 1,5-dinitronaphthalene proportion
Procédé de production d' un mélange d' isomères de dinitronaphthalène à proportion élevée en 1,5-dinitronaphthalène

(30) Priorität: 05.03.2002 DE 10209644
(43) Veröffentlichungstag der Anmeldung: 10.09.2003
(73) Patentinhaber: Bayer MaterialScience AG, 51368 Leverkusen (DE)
(72) Erfinder: Brandt, Matthias, Dr., 40589 Düsseldorf (DE); Klein, Stephan, Dr., 40822 Mettmann (DE); Wegener, Gerhard, Dr., 40822 Mettmann (DE)

(56) Entgegenhaltungen:
- EP-A- 1 004 570
- WO-A-96/36587
- DE-C- 19 739 202
- GB-A- 1 499 699
- DATABASE WPI Section Ch, Week 199748 Derwent Publications Ltd., London, GB; Class E14, AN 1997-524733 XP002244525 & RU 2 079 482 C (AS SIBE CATALYSIS INST), 20. Mai 1997 (1997-05-20)
- DATABASE WPI Section Ch, Week 199633 Derwent Publications Ltd., London, GB; Class A41, AN 1996-329446 XP002244526 & JP 08 151353 A (MITSUI TOATSU CHEM INC), 11. Juni 1996 (1996-06-11)

## Beschreibung

Die Erfindung betrifft die Herstellung eines Dinitronaphthalin-Isomerengemisches mit hohem Anteil an 1,5-Dinitronaphthalin durch Nitrierung von Naphthalin, 1-Nitronaphthalin oder einem Nitronaphthalin-Rohgemisch. 1,5-Dinitronaphthalin ist eine Schlüsselverbindung für die Herstellung von 1,5-Diaminonaphthalin. Dieses ist u. a. die Ausgangsverbindung zur Herstellung von 1,5-Diisocyanatonaphthalin (Handelsname: Desmodur 15® ). 1,5-Diisocyanatonaphthalin wird als Isocyanatkomponente bei der Polyurethanherstellung eingesetzt.

Die Herstellung von nitrierten Aromaten ist seit langem bekannt (G. A. Olah et al., Nitration: Methods and Mechanisms, VCH, New York, 1989). Seit Jahrzehnten werden entsprechende Nitroaromaten durch Nitrierung mit einer Mischung aus Schwefel- und Salpetersäure (sogenannte Misch- oder Nitriersäure) technisch hergestellt.

Die Mononitrierung von Naphthalin liefert ein Isomerengemisch von 1-Nitronaphthalin und 2-Nitronaphthalin im Verhältnis von etwa 95 : 5. Die direkte Dinitrierung von Naphthalin wie auch die Weiternitrierung von 1-Nitronaphthalin führen zu 1,5-Dinitronaphthalin und 1,8-Dinitronaphthalin im Verhältnis von etwa 1: 2 (Houben-Weyl: Methoden der Organischen Chemie, 4. Aufl., 1971, Band X/1, S. 492-495). In geringerem Umfang (etwa 5 %) werden auch andere Dinitronaphthalin-Isomere gebildet, z.B. 1,6- und 1,7-Dinitronaphthalin. Die ungünstige Selektivität der Reaktionen führt also dazu, dass bei der Herstellung von 1,5-Dinitronaphthalin vorzugsweise ein hoher und unerwünschter Anteil an 1,8-Dinitronaphthalin entsteht.

In DE-OS-11 50 965 wird die Herstellung von Dinitronaphthalinen ausgehend von 1-Nitronaphthalin beschrieben. Eine Erhöhung der Selektivität zugunsten des gewünschten 1,5-Dinitronaphthalin wird durch schnelles und intensives Vermischen des in Schwefelsäure gelösten 1-Nitronaphthalins mit Nitriersäure erreicht. Nachteil dieses Verfahrens ist die erhebliche Menge an Schwefelsäure und deren aufwendige und kostenintensive Aufarbeitung. Außerdem können bei diesem Verfahren erhebliche Mengen an trinitrierten Produkten entstehen, die sowohl die 1,5-Dinitronaphthalin-Ausbeute deutlich mindern, als auch in Bezug auf die Sicherheitstechnik als kritisch anzusehen sind, insbesondere bei der im genannten Stand der Technik beschriebenen adiabatischen Reaktionsführung.

In WO-99/12886 wird - ausgehend von Nitronaphthalin - ein Verfahren zur Herstellung eines Dinitronaphthalin-Isomerengemisches mit hohem Anteil an 1,5-Dinitronaphthalin beschrieben. Hierbei findet die Umsetzung des Nitronaphthalins mit Salpetersäure in Nitroalkanen oder Sulfolan als Lösemittel statt. Bei diesem Verfahren werden aber Produktgemische erhalten, die noch erhebliche Anteile des nicht umgesetzten Eduktes 1-Nitronaphthalin enthalten. Ein weiterer Nachteil liegt in den geringen Ausbeuten an 1,5-Dinitronaphthalin, das nur zu maximal 28,3 % in den Produktgemischen enthalten ist.

In WO-99/12887 wird - ebenfalls ausgehend von Nitronaphthalin - ein Verfahren zur Herstellung eines Dinitronaphthalin-Isomerengemisches mit hohem Anteil an 1,5-Dinitronaphthalin beschrieben. Hierbei findet die Umsetzung des Nitronaphthalins mit Salpetersäure in Gegenwart eines festen, perfluorierten, stark sauren Ionenaustauschers statt. Generell hat dieses Verfahren den Nachteil, dass das entstandene Dinitronaphthalin-Isomerengemisch durch Extraktion mit Dioxan bei 90°C vom Katalysator abgetrennt werden muss. Das Dioxan muss anschließend durch einen zusätzlichen Destillationsschritt entfernt werden. Ein weiterer Nachteil des Verfahrens besteht darin, dass zur Erreichung hoher Umsätze des Eduktes 1-Nitronaphthalin die Salpetersäure im großen Überschuss (6 bis 8 Äquivalente) zugesetzt werden muss. Ebenfalls nachteilig ist der Umstand, dass die Produktgemische mit relativ hohem Anteil (> 30 %) an 1,5-Dinitronaphthalin stets noch höhere Anteile an 1,8-Dinitronaphthalin enthalten.

DE-OS-24 53 529 beschreibt die Herstellung von Dinitronaphthalinen durch Nitrierung von Naphthalin oder 1-Nitronaphthalin mit Salpetersäure in organischen Lösungsmitteln, beispielsweise Dichlorethan, unter azeotroper Entfernung des Reaktionswassers. Dieses Verfahren liefert Dinitronaphthalin in hohen Ausbeuten, ohne jedoch das Isomerenverhältnis zu beeinflussen.

WO-94/19310 beschreibt Nitrierungen von Aromaten an teilweise mit Schwermetallen dotierten Aluminiumsilikaten, sogenannten Claycops, als festen Katalysatoren. Die nach diesem Verfahren durchgeführte Nitrierung von Naphthalin liefert Dinitronaphthalin in hoher Ausbeute, allerdings mit einem Isomerenverhältnis wie bei klassischen Nitrierungen mit Mischsäure.

In DE-A1-199 58 389 wird ein Verfahren zur Herstellung eines Dinitronaphthalin-Isomerengemisches mit erhöhtem Anteil an 1,5-Dinitronaphthalin beschrieben, bei dem Naphthalin mit Salpetersäure in Gegenwart mindestens einer ionischen Flüssigkeit durchgeführt wird. Solche ionischen Flüssigkeiten sind aber sehr teuer und daher für den Einsatz im industriellen Maßstab ungeeignet. Ein weiterer Nachteil des Verfahrens liegt darin, dass sehr hohe Überschüsse an Salpetersäure (8 bis 22 Äquivalente pro einzuführender Nitrogruppe) eingesetzt werden. Desweiteren liegt der Isomerenanteil des unerwünschten 1,8-Dinitronaphthalins mit 50 bis 53 % stets deutlich über dem des 1,5-Dinitronaphthalins (36,5 bis 39 %).

Aufgabe der vorliegenden Erfindung ist daher, ein einfaches und wirtschaftliches Verfahren zur Herstellung eines Dinitronaphthalin-Isomerengemisches bereitzustellen, mit dem ein Gemisch von Dinitronaphthalinen in hohen Ausbeuten und mit einem großen Anteil an 1,5-Dinitronaphthalin erhalten werden kann.

Es wurde gefunden, dass bei Einsatz von Zeolithen als Katalysator bei der Nitrierung von Naphthalin und/oder 1-Nitronaphthalin mit Salpetersäure eine Verschiebung des Isomerenverhältnisses hin zum 1,5-Dinitronaphthalin möglich ist.

Gegenstand der Erfindung ist ein Verfahren zur Herstellung eines Dinitronaphthalin-Isomerengemisches, bei dem man Naphthalin und/oder 1-Nitronaphthalin mit Salpetersäure in Gegenwart von Zeolithen umsetzt.

Die erfindungsgemäß hergestellten Dinitronaphthalin-Isomerengemische enthalten einen überraschend hohen Anteil an 1,5-Dinitronaphthalin. Der erzielbare Anteil an 1,5-Dinitronaphthalin liegt dabei bei ca. 60 Gew.-% bezogen auf das Gemisch aus 1,5-Dinitronaphthalin und 1,8-Dinitronaphthalin. Der Gehalt an anderen Nebenprodukten, insbesondere weiteren Dinitronaphthalin-Isomeren und höher nitrierten Produkten, ist gering.

In dem Verfahren kann als Ausgangsprodukt Naphthalin,/reines 1-Nitronaphthalin oder auch ein Nitronaphthalin-Rohgemisch, wie es bei der Mononitrierung von Naphthalin als Rohprodukt erhalten wird, eingesetzt werden.

Das erfindungsgemäße Verfahren wird in Gegenwart mindestens eines Zeolithen durchgeführt. Dabei können auch Mischungen verschiedener Zeolithe eingesetzt werden.

Zeolithe sind von ihrer Grundstruktur her kristalline Alumosilikate, die aus einem Netzwerk von SiO₄- bzw. AlO₄-Tetraedern aufgebaut sind. Die einzelnen Tetraeder sind mit Sauerstoffbrücken über die Ecken untereinander verknüpft und bilden ein räumliches Netzwerk, das gleichmäßig von Kanälen und Hohlräumen durchzogen ist. Als Ausgleich für die negative Ladung des Gitters sind austauschbare Kationen eingelagert. Dabei kann Aluminium teilweise durch andere Elemente wie beispielsweise B, Ga, In, Fe, Cr, V, As, Sb oder Be ersetzt werden. Weiterhin kann Silicium durch andere vierwertige Elemente ersetzt sein, wie beispielsweise Ge, Ti, Zr oder Hf. Als austauschbare Kationen können die Zeolithe beispielsweise außerdem H, NH₄, Li, Na, K, Mg, Ca, Cu, Zn, Seltene Erdmetalle, Ti, Zr, Sn(IV), Cr(III), Fe(II), Mn(II), Co oder Ni enthalten.

Es werden Zeolithe der Strukturtypen MFI, MOR, BEA, FAU, MEL, EMT, MTW, LTL, MWW, RHO, FER oder HEU (gemäß der Strukturklassifizierung aus W. M. Meier, D. H. Olson, Ch. Baerlocher, Atlas of Zeolite Structure Types, 4^{th} Edition, Elsevier, London, 1996) in der sauren H⁺-Form verwendet. Im einzelnen kommen insbesondere die Zeolithe H-beta, H-Y, H-Mordenit und H-ZSM-5 in Betracht. Besonders bevorzugt werden Zeolithe vom Typ H-Y eingesetzt.

Zeolithe in der sauren H⁺-Form und ihre Herstellung sind in der Literatur eingehend beschrieben (R. Szostak, Handbook of Molecular Sieves, Van Nostrand Reinhold, New York, 1992).

Die Zeolithe können ohne Vorbehandlung eingesetzt werden oder aber gegebenenfalls auch vorbehandelt werden. Die Vorbehandlung kann beispielsweise durch Calcinieren bei einer Temperatur zwischen 200 und 700°C, bevorzugt 300 bis 600°C erfolgen. Die Calcinierung kann beispielsweise über einen Zeitraum von 1 bis 24 Stunden durchgeführt werden. Nach der Calcinierung wird der Zeolith an Luft oder vorzugsweise unter Inertatmosphäre wie beispielsweise Stickstoff-, Helium- oder Argonatmosphäre abgekühlt und anschließend für die Nitrierung eingesetzt.

Es können die Zeolithe in Form von Pulvern, Granulaten, Partikeln, Kugeln, Tabletten, Strangpresslingen oder Extrudaten eingesetzt werden. Weiterhin können die Zeolithe in eine anorganische Matrix eingebettet sein, welche bevorzugt inert ist. Geeignete anorganische Matrixmaterialien sind beispielsweise Siliciumdioxid, Siliciumcarbid, Aluminiumoxid, synthetische poröse Materialien oder Ton. Desweiteren können die Zeolithe auch auf Trägerstrukturen aufgebracht sein, welche bevorzugt inert sind. Geeignete Trägerstrukturen sind beispielsweise keramische Monolithe, keramische Wabenkörper, keramische Schäume, keramische strukturierte Packungen, metallische Monolithe, metallische Wabenkörper, metallische Schäume, Drahtgestricke, Metallträger mit Kreuzkanalstruktur, metallische strukturierte Packungen und Taschenpackungen aus Drahtgewebe.

Die Zeolithe können bei Auftreten von Aktivitätsverlusten beispielsweise durch Waschen, Säurebehandlung oder Calcinieren regeneriert und anschließend wieder in dem erfindungsgemäßen Verfahren eingesetzt werden.

Die Salpetersäure wird bevorzugt als wässrige Lösung eingesetzt. Die Konzentration der eingesetzten Salpetersäure liegt dabei bevorzugt zwischen 40 und 100 Gew.-%, besonders bevorzugt zwischen 50 und 100 Gew.-%, ganz besonders bevorzugt zwischen 60 und 99 Gew.-%.

Die Salpetersäure kann im Überschuss, in der stöchiometrisch benötigten Menge oder im Unterschuss eingesetzt werden. Ein Salpetersäure-Überschuss erleichtert das Erreichen eines hohen Naphthalin- und/oder 1-Nitronaphthalin-Umsatzes; die nicht umgesetzte Salpetersäure kann abgetrennt und in den Prozess zurückgeführt werden. Ein Salpetersäure-Unterschuss erleichtert das Erreichen eines hohen bis vollständigen Salpetersäure-Umsatzes; nicht umgesetztes Naphthalin und/oder 1-Nitronaphthalin kann abgetrennt und in den Prozess zurückgerührt werden.

Bevorzugt wird pro Mol Naphthalin 0,2 bis 40 Mol, besonders bevorzugt 0,4 bis 20 Mol Salpetersäure und/oder pro Mol 1-Nitronaphthalin bevorzugt 0,1 bis 20 Mol, besonders bevorzugt 0,2 bis 10 Mol Salpetersäure eingesetzt.

In einer möglichen Ausführungsform des Verfahrens wird die Salpetersäure in einer Mischung mit Schwefelsäure und/oder Phosphorsäure eingesetzt.

Die gegebenenfalls eingesetzte Schwefelsäure wird bevorzugt als wässrige Lösung eingesetzt. Die Konzentration der Schwefelsäure liegt dabei bevorzugt zwischen 85 und 100 Gew.-%, besonders bevorzugt zwischen 90 und 100 Gew.-% und ganz besonders bevorzugt zwischen 95 und 100 Gew.-%.

Die gegebenenfalls eingesetzte Phosphorsäure wird bevorzugt als wässrige Lösung eingesetzt. Die Konzentration der Phosphorsäure liegt dabei bevorzugt zwischen 50 und 99 Gew.-%, besonders bevorzugt zwischen 65 und 99 Gew.-% und ganz besonders bevorzugt zwischen 85 und 99 Gew.-%.

Bei Einsatz einer Mischung aus Salpetersäure und Schwefelsäure und/oder Phosphorsäure besteht diese Mischung vorzugsweise aus 1 bis 20 Gewichtsteilen Salpetersäure und 1 Gewichtsteil Schwefelsäure und/oder Phosphorsäure, besonders bevorzugt aus 1 bis 10 Gewichtsteilen Salpetersäure und 1 Gewichtsteil Schwefelsäure und/oder Phosphorsäure, ganz besonders bevorzugt aus 1 bis 5 Gewichtsteilen Salpetersäure und 1 Gewichtsteil Schwefelsäure und/oder Phosphorsäure.

Vorzugsweise wird das Verfahren bei Temperaturen zwischen 20 und 160°C, besonders bevorzugt bei Temperaturen zwischen 40 und 120°C und ganz besonders bevorzugt bei Temperaturen zwischen 50 und 100°C durchgeführt.

Das Verfahren kann ohne Lösemittel oder in Gegenwart eines Lösemittels durchgeführt werden. Bevorzugt wird das Verfahren in Gegenwart eines organischen Lösemittels durchgeführt. Geeignete Lösemittel sind alle unter den Bedingungen der Nitrierung stabilen Lösemittel, beispielsweise Kohlenwasserstoffe, halogenierte Kohlenwasserstoffe, Carbonsäuren, Nitroalkane, Nitroaromaten, Sulfolan und Dimethylsulfoxid. Bevorzugt werden als Lösemittel n-Alkane mit 5 bis 16 Kohlenstoffatomen, Cycloalkane mit 5 bis 8 Kohlenstoffatomen, Ligroin, perfluorierte Kohlenwasserstoffe, Dichlormethan, Chloroform, Tetrachlorkohlenstoff, 1,2-Dichlorethan, 1,2-Dichlorpropan, 1,2-Dichlorbenzol, Essigsäure, Propionsäure, Nitromethan, Nitroethan, Nitrobenzol, Dinitrobenzol, Sulfolan und Dimethylsulfoxid eingesetzt. Besonders bevorzugt werden als Lösemittel n-Hexan, n-Heptan, Cyclohexan, Ligroin, Dichlormethan, Chloroform, 1,2-Dichlorethan, 1,2-Dichlorpropan, Essigsäure, Nitromethan, Nitroethan und Sulfolan eingesetzt.

Die Menge an zugesetztem organischen Lösemittel, bezogen auf die eingesetzte Menge an Naphthalin oder Nitronaphthalin, liegt bevorzugt zwischen 10 und 10000 Gew.-%, besonders bevorzugt zwischen 50 und 5000 Gew.-%, ganz besonders bevorzugt zwischen 100 und 5000 Gew.-%.

In dem Verfahren können auch Mischungen verschiedener Lösemittel eingesetzt werden.

Das Verfahren kann kontinuierlich, teilkontinuierlich oder diskontinuierlich bei reduziertem, normalem oder erhöhtem Druck durchgeführt werden. Es ist auch möglich, simultan zur Reaktion eine oder mehrere Komponenten aus dem Reaktionsgemisch abzutrennen, beispielsweise durch Strippung, Destillation oder Membrantrennverfahren. Bevorzugt wird das Verfahren teilkontinuierlich bei normalem Druck durchgeführt. Besonders bevorzugt wird das Verfahren derart durchgeführt, dass zu einer Mischung aus Naphthalin oder Nitronaphthalin, Zeolith und gegebenenfalls organischem Lösemittel bei der gewünschten Reaktiönstemperatur die Salpetersäure zudosiert wird, wobei der Zeolith vorzugsweise in einer Menge von 1 bis 1000 Gew.-%, besonders bevorzugt 5 bis 750 Gew.-% bezogen auf die Menge an Naphthalin oder Nitronaphthalin eingesetzt wird.

Das Verfahren kann auf bekannte Weise in verschiedenen Reaktoren durchgeführt werden, die sich für feststoffkatalysierte Flüssigphasenreaktionen eignen, beispielsweise Rührreaktoren, Festbettreaktoren und Fließbettreaktoren. Dabei ist auch eine Kreislauffahrweise möglich. Bevorzugt wird das Verfahren in Rührreaktoren durchgeführt.

Bevorzugt wird das Verfahren derart durchgeführt, dass Naphthalin oder 1-Nitronaphthalin, der Zeolithkatalysator und gegebenenfalls organisches Lösemittel vermischt und auf Reaktionstemperatur erwärmt werden, und dann die einzusetzende Säure auf einmal oder über einen längeren Zeitraum kontinuierlich oder in Portionen zugegeben wird. Um sicherzustellen, dass die Reaktion vollständig abläuft, wird die Umsetzung vorzugsweise unter guter Durchmischung des Reaktionsansatzes, beispielsweise durch intensives Rühren, durchgeführt. Die Reaktionsdauer liegt üblicherweise zwischen 5 Minuten und 24 Stunden, bevorzugt zwischen 30 Minuten und 18 Stunden. Die Aufarbeitung des Reaktionsgemisches kann auf dem Fachmann bekannte Weise erfolgen.

Die Abtrennung des Zeolith-Katalysators vom Reaktionsgemisch nach beendeter Umsetzung kann beispielsweise durch Filtration, Sedimentation oder Zentrifugation erfolgen. Wird der Zeolith als fest angeordneter Katalysator in einem Festbettreaktor eingesetzt, der vom übrigen Reaktionsgemisch durchströmt wird, so ist eine gesonderte Abtrennung nicht erforderlich.

Nicht umgesetztes Naphthalin oder 1-Nitronaphthalin, überschüssige Salpetersäure und gegebenenfalls das Lösemittel können beispielsweise durch Phasentrennung, Destillation oder fraktionierte Kristallisation vom gebildeten Dinitronaphthalin-Isomerengemisch abgetrennt und in den Prozess zurückgeführt werden.

Das Dinitronaphthalin-Isomerengemisch kann beispielsweise durch fraktionierte Kristallisation in die isomeren Dinitronaphthaline getrennt werden. Derartige Isomerentrennungen, beispielsweise mit Dimethylformamid oder Dichlorethan als Lösemittel, sind allgemein bekannt (Houben-Weyl: Methoden der Organischen Chemie, 4. Aufl., 1971, Band X/1, S. 494).

Gegenstand der Erfindung ist auch ein Verfahren zur Herstellung von 1,5-Diaminonaphthalin, bei dem man Naphthalin und/oder 1-Nitronaphthalin mit Salpetersäure in Gegenwart von den im Anspruch 1 beanspruchten Zeolithen umsetzt, und anschließend das entstandene 1,5-Dinitronaphthalin zum 1,5-Diaminonaphthalin hydriert.

Gegenstand der Erfindung ist auch ein Verfahren zur Herstellung von 1,5-Diisocyanatonaphthalin, bei dem man Naphthalin und/oder 1-Nitronaphthalin mit Salpetersäure in Gegenwart von Zeolithen umsetzt, anschließend das entstandene 1,5-Dinitronaphthalin zum 1,5-Diaminonaphthalin hydriert, und anschließend das entstandene 1,5-Diaminonaphthalin zum 1,5-Diisocyanatonaphthalin phosgeniert.

Die Hydrierung von 1,5-Dinitronaphthalin zum 1,5-Diaminonaphthalin und die Phosgenierung des 1,5-Diaminonaphthalins zum 1,5-Diisocyanatonaphthalin erfolgt dabei auf die nach dem Stand der Technik bekannte Weise (Houben-Weyl: Methoden der Organischen Chemie, 4. Aufl., Band XI/1, S. 400 - 401 (1957), und Houben-Weyl: Methoden der Organischen Chemie, 4. Aufl., Band E 4, S. 741 - 748 (1983)).

### Beispiele

Die in den folgenden Beispielen eingesetzten Zeolith-Katalysatoren wurden vor den Reaktionen mindestens 3 Stunden bei 500°C calciniert.

Die Analyse der Zusammensetzung der entstandenen Gemische erfolgte gaschromatographisch, wobei die Quantifizierung mit Hilfe eines inneren Standards (n-Hexadecan) erfolgte.

In allen folgenden Beispielen wurden Trinitronaphthaline gar nicht oder allenfalls in sehr kleinen Mengen gebildet. Andere Neben- oder Folgeprodukte wurden in keinem Fall beobachtet.

### Beispiel 1

### Nitrierung von 1-Nitronaphthalin mit Salpetersäure in Gegenwart von 1,2-Dichlorethan und Zeolith H-Y

11,0 g 1,2-Dichlorethan, 0,6 g 1-Nitronaphthalin und 2,0 g Zeolith H-Y der Firma PQ (Pulver, SiO₂/Al₂O₃ = 60) wurden vorgelegt und auf 73°C erwärmt. Dann wurden 0,60 g 99 gew.-%ige Salpetersäure auf einmal zugegeben. Anschließend wurde der Ansatz 3 Stunden bei 73°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 2 (Vergleichsbeispiel)

### Nitrierung von 1-Nitronaphthalin mit Salpetersäure in Gegenwart von 1,2-Dichlorethan

11,0 g 1,2-Dichlorethan und 0,6 g 1-Nitronaphthalin wurden vorgelegt und auf 73°C erwärmt. Dann wurden 0,60 g 99 gew.-%ige Salpetersäure auf einmal zugegeben. Anschließend wurde der Ansatz 3 Stunden bei 73°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 3

### Nitrierung von 1-Nitronaphthalin mit Salpetersäure in Gegenwart von 1,2-Dichlorethan und Zeolith H-Y

10,0 g 1,2-Dichlorethan, 0,60 g 1-Nitronaphthalin und 1,5 g Zeolith H-Y der Firma PQ (Pulver, SiO₂/Al₂O₃ = 60) wurden vorgelegt und auf 80°C erwärmt. Dann wurden 0,30 g 99 gew.-%ige Salpetersäure auf einmal zugegeben. Anschließend wurde der Ansatz 22 Stunden bei 80°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 4

### Nitrierung von 1-Nitronaphthalin mit Salpetersäure in Gegenwart von 1,2-Dichlorethan und Zeolith H-Y

10,0 g 1,2-Dichlorethan, 0,30 g 1-Nitronaphthalin und 1,5 g Zeolith H-Y der Firma PQ (Pulver, SiO₂/Al₂O₃ = 60) wurden vorgelegt und auf 80°C erwärmt. Dann wurden 0,30 g 99 gew.-%ige Salpetersäure auf einmal zugegeben. Anschließend wurde der Ansatz 50 Minuten bei 80°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 5

### Nitrierung von 1-Nitronaphthalin mit Salpetersäure in Gegenwart von 1,2-Dichlorethan und Zeolith H-Y

10,0 g 1,2-Dichlorethan, 0,50 g 1-Nitronaphthalin und 1,5 g Zeolith H-Y der Firma PQ (Pulver, SiO₂/Al₂O₃ = 30) wurden vorgelegt und auf 70°C erwärmt. Dann wurden 0,60 g 99 gew.-%ige Salpetersäure innerhalb von 30' Minuten stufenweise zugegeben. Anschließend wurde der Ansatz 210 Minuten bei 70°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 6 (Vergleichsbeispiel)

### Nitrierung von 1-Nitronaphthalin mit Salpetersäure in Gegenwart von 1,2-Dichlorethan

10,0 g 1,2-Dichlorethan und 0,50 g 1-Nitronaphthalin wurden vorgelegt und auf 70°C erwärmt. Dann wurden 0,60 g 99 gew.-%ige Salpetersäure innerhalb von 30 Minuten stufenweise zugegeben. Anschließend wurde der Ansatz 210 Minuten bei 70°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 7

### Nitrierung von 1-Nitronaphthalin mit Salpetersäure in Gegenwart von Chloroform und Zeolith H-Y

10,0 g Chloroform, 0,40 g 1-Nitronaphthalin und 1,5 g Zeolith H-Y der Firma PQ (Pulver, SiO₂/Al₂O₃ = 30) wurden vorgelegt und auf 61°C erwärmt. Dann wurden 0,90 g 99 gew.-%ige Salpetersäure innerhalb von 50 Minuten stufenweise zugegeben. Anschließend wurde der Ansatz 190 Minuten bei 61°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 8 (Vergleichsbeispiel)

### Nitrierung von 1-Nitronaphthalin mit Salpetersäure in Gegenwart von Chloroform

10,0 g Chloroform und 0,40 g 1-Nitronaphthalin wurden vorgelegt und auf 61°C erwärmt. Dann wurden 0,90 g 99 gew.-%ige Salpetersäure innerhalb von 50 Minuten stufenweise zugegeben. Anschließend wurde der Ansatz 190 Minuten bei 61°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 9

### Nitrierung von 1-Nitronaphthalin mit Salpetersäure in Gegenwart von n-Heptan und Zeolith H-Y

6,5 g n-Heptan, 0,50 g 1-Nitronaphthalin und 1,5 g Zeolith H-Y der Firma PQ (Pulver, SiO₂/Al₂O₃ = 12) wurden vorgelegt und auf 60°C erwärmt. Dann wurden 0,45 g 99 gew.-%ige Salpetersäure innerhalb von 20 Minuten stufenweise zugegeben. Anschließend wurde der Ansatz 160 Minuten bei 60°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 10

### Nitrierung von 1-Nitronaphthalin mit Salpetersäure in Gegenwart von 1,2-Dichlorethan und Sulfolan und Zeolith H-Y

10,0 g 1,2-Dichlorethan, 1,5 g Sulfolan, 0,60 g 1-Nitronaphthalin und 1,5 g Zeolith H-Y der Firma PQ (Pulver, SiO₂/Al₂O₃ = 30) wurden vorgelegt und auf 80°C erwärmt. Dann wurden 0,60 g 99 gew.-%ige Salpetersäure innerhalb von 30 Minuten stufenweise zugegeben. Anschließend wurde der Ansatz 180 Minuten bei 80°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 11

### Nitrierung von 1-Nitronaphthalin mit Salpetersäure in Gegenwart von 1,2-Dichlorethan und Zeolith H-Y

10,0 g 1,2-Dichlorethan, 0,20 g 1-Nitronaphthalin und 1,5 g Zeolith H-Y der Firma PQ (Pulver, SiO₂/Al₂O₃ = 30) wurden vorgelegt und auf 83°C erwärmt. Dann wurden 0,15 g 99 gew.-%ige Salpetersäure auf einmal zugegeben. Anschließend wurde der Ansatz 1 Stunde bei 83°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 12

### Nitrierung von 1-Nitronaphthalin mit Salpetersäure in Gegenwart von 1,2-Dichlorethan und Zeolith H-Y

10,0 g 1,2-Dichlorethan, 0,20 g 1-Nitronaphthalin und 1,5 g Zeolith H-Y der Firma PQ (Pulver, SiO₂/Al₂O₃ = 30) wurden vorgelegt und auf 50°C erwärmt. Dann wurden 0,23 g 99 gew.-%ige Salpetersäure auf einmal zugegeben. Anschließend wurde der Ansatz 4 Stunden bei 50°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 13

### Nitrierung von 1-Nitronaphthalin mit Salpetersäure in Gegenwart von 1,2-Dichlorethan und Zeolith H-Y

10,0 g 1,2-Dichlorethan, 0,30 g 1-Nitronaphthalin und 1,5 g Zeolith H-Y der Firma PQ (Pulver, SiO₂/Al₂O₃ = 60) wurden vorgelegt und auf 80°C erwärmt. Dann wurden 0,30 g 99 gew.-%ige Salpetersäure auf einmal zugegeben. Anschließend wurde der Ansatz 20 Minuten bei 80°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 14

### Nitrierung von 1-Nitronaphthalin mit Salpetersäure in Gegenwart von Nitromethan und Zeolith H-Y

10,0 g Nitromethan, 0,60 g 1-Nitronaphthalin und 1,5 g Zeolith H-Y der Firma PQ (Pulver, SiO₂/Al₂O₃ = 30) wurden vorgelegt und auf 78°C erwärmt. Dann wurden 0,60 g 99 gew.-%ige Salpetersäure innerhalb von 30 Minuten stufenweise zugegeben. Anschließend wurde der Ansatz 210 Minuten bei 78°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 15 (Vergleichsbeispiel)

### Nitrierung von 1-Nitronaphthalin mit Salpetersäure in Gegenwart von Nitromethan

10,0 g Nitromethan und 0,60 g 1-Nitronaphthalin wurden vorgelegt und auf 78°C erwärmt. Dann wurden 0,90 g 99 gew.-%ige Salpetersäure innerhalb von 50 Minuten stufenweise zugegeben. Anschließend wurde der Ansatz 190 Minuten bei 78°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 16

### Nitrierung von 1-Nitronaphthalin mit Salpetersäure in Gegenwart von Nitromethan und 1,2-Dichlormethan und Zeolith H-Y

5,0 g Nitromethan, 5,0 g 1,2-Dichlorethan, 0,60 g 1-Nitronaphthalin und 1,5 g Zeolith H-Y der Firma PQ (Pulver, SiO₂/Al₂O₃ = 30) wurden vorgelegt und auf 78°C erwärmt. Dann wurden 0,60 g 99 gew.-%ige Salpetersäure innerhalb von 30 Minuten stufenweise zugegeben. Anschließend wurde der Ansatz 210 Minuten bei 78°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 17

### Nitrierung von 1-Nitronaphthalin mit Salpetersäure in Gegenwart von Nitromethan und Zeolith H-Y

10,0 g Nitromethan, 0,80 g 1-Nitronaphthalin und 0,4 g Zeolith H-Y der Firma PQ (Pulver, SiO₂/Al₂O₃ = 30) wurden vorgelegt und auf 80°C erwärmt. Dann wurden 1,20 g 99 gew.-%ige Salpetersäure innerhalb von 70 Minuten stufenweise zugegeben. Anschließend wurde der Ansatz 170 Minuten bei 80°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 18

### Nitrierung von 1-Nitronaphthalin mit Salpetersäure in Gegenwart von Nitroethan und Zeolith H-Y

10,0 g Nitroethan, 0,50 g 1-Nitronaphthalin und 1,5 g Zeolith H-Y der Firma PQ (Pulver, SiO₂/Al₂O₃ = 80) wurden vorgelegt und auf 78°C erwärmt. Dann wurden 0,75 g 99 gew.-%ige Salpetersäure innerhalb von 40 Minuten stufenweise zugegeben. Anschließend wurde der Ansatz 200 Minuten bei 78°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

### Beispiel 19 (Vergleichsbeispiel)

### Nitrierung von 1-Nitronaphthalin mit Salpetersäure in Gegenwart von Nitroethan

10,0 g Nitroethan und 0,50 g 1-Nitronaphthalin wurden vorgelegt und auf 78°C erwärmt. Dann wurden 0,75 g 99 gew.-%ige Salpetersäure innerhalb von 40 Minuten stufenweise zugegeben. Anschließend wurde der Ansatz 200 Minuten bei 78°C gerührt. Die Aufarbeitung erfolgte durch Zugabe von 10 mL vollentsalztem Wasser und anschließende Extraktion mit 60 mL Dichlormethan. Das Ergebnis ist in Tabelle 1 aufgeführt.

**Tabelle 1**

| **Beispiel** | **HNO₃/1-NN (mol/mol)** | **U (1-NN) (%)** | **R (1,5- DNN) (%)** | **R (1,8- DNN) (%)** | **1,5-DNN/ 1,8-DNN (mol/mol)** | **A (1,5- DNN) (%)** |
|---|---|---|---|---|---|---|
| 1 | 2,7 | 74,5 | 44,2 | 44,4 | 0,99 | 32,7 |
| 2 (V) | 2,7 | 38,8 | 25,5 | 70,7 | 0,36 | 9,9 |
| 3 | 1,35 | 86,5 | 44,1 | 45,2 | 0,98 | 38,1 |
| 4 | 2,7 | 98,5 | 46,2 | 44,3 | 1,04 | 45,5 |
| 5 | 3,3 | 92,5 | 45,2 | 43,9 | 1,03 | 41,7 |
| 6 (V) | 3,3 | 77,6 | 25,8 | 70,6 | 0,37 | 20,0 |
| 7 | 6,1 | 96,0 | 40,5 | 49,7 | 0,82 | 38,7 |
| 8 (V) | 6,1 | 93,3 | 26,3 | 70,7 | 0,37 | 24,5 |
| 9 | 2,4 | 65,4 | 44,1 | 44,9 | 0,98 | 28,5 |
| 10 | 2,7 | 58,2 | 44,9 | 47,7 | 0,95 | 26,2 |
| 11 | 2,0 | 100 | 48,6 | 41,2 | 1,18 | 48,6 |
| 12 | 3,1 | 100 | 50,3 | 40,8 | 1,23 | 50,3 |
| 13 | 2,7 | 78,0 | 48,7 | 41,8 | 1,16 | 37,9 |
| 14 | 2,7 | 99,0 | 57,9 | 34,8 | 1,66 | 57,3 |
| 15 (V) | 4,1 | 83,3 | 36,7 | 57,2 | 0,64 | 30,6 |
| 16 | 2,7 | 91,6 | 54,0 | 38,1 | 1,42 | 49,5 |
| 17 | 4,1 | 99,2 | 48,6 | 44,0 | 1,11 | 48,2 |
| 18 | 4,1 | 100 | 50,4 | 44,0 | 1,14 | 50,4 |
| 19 (V) | 4,1 | 73,6 | 35,4 | 59,3 | 0,60 | 26,1 |

| | | | | | | |
|---|---|---|---|---|---|---|
| (V): Vergleichsbeispiel 1-NN: 1-Nitronaphthalin 1,5-DNN: 1,5-Dinitronaphthalin 1,8-DNN: 1,8-Dinitronaphthalin U: Umsatz R: Regioselektivität = Isomerenanteil A: Ausbeute | | | | | | |

### Beispiel 20

### Nitrierung von Naphthalin mit Salpetersäure in Gegenwart von 1,2-Dichlorethan und Zeolith H-Y

10,0 g 1,2-Dichlorethan, 0,44 g Naphthalin und 1,5 g Zeolith H-Y der Firma Degussa (zu Pulver zermahlenes Extrudat, SiO₂/Al₂O₃ = 55) wurden vorgelegt und auf 80°C erwärmt. Dann wurden 0,90 g 99 gew.-%ige Salpetersäure innerhalb von 40 Minuten stufenweise zugegeben. Anschließend wurde der Ansatz 200 Minuten bei 80°C gerührt. Die Aufarbeitung erfolgte durch Zugabe von 10 mL vollentsalztem Wasser und anschließende Extraktion mit 60 mL Dichlormethan.
Ergebnisse:
- Umsatz an Naphthalin: 100%
- Ausbeute an 1-Nitronaphthalin: 1,0 %
- Regioselektivität zum 1,5-Dinitronaphthalin: 38,3 %
- Regioselektivität zum 1,8-Dinitronaphthalin: 46,8 %
- Molverhältnis 1,5-/1,8-Dinitronaphthalin: 0,82

### Beispiel 21 (Vergleichsbeispiel)

### Nitrierung von Naphthalin mit Salpetersäure in Gegenwart von 1,2-Dichlorethan

10,0 g 1,2-Dichlorethan und 0,44 g Naphthalin wurden vorgelegt und auf 80°C erwärmt. Dann wurden 0,90 g 99 gew.-%ige Salpetersäure innerhalb von 20 Minuten stufenweise zugegeben. Anschließend wurde der Ansatz 220 Minuten bei 80°C gerührt. Die Aufarbeitung erfolgte durch Zugabe von 10 mL vollentsalztem Wasser und anschließende Extraktion mit 60 mL Dichlormethan.
Ergebnisse:
- Umsatz an Naphthalin: 100 %
- Ausbeute an 1-Nitronaphthalin: 62,2 %
- Regioselektivität zum 1,5-Dinitronaphthalin: 25,2 %
- Regioselektivität zum 1,8-Dinitronaphthalin: 65,7%
- Molverhältnis 1,5-/1,8-Dinitronaphthalin: 0,38

### Beispiel 22

### Nitrierung von 1-Nitronaphthalin mit Salpetersäure in Gegenwart von Sulfolan und Zeolith H-Y

302 g Sulfolan, 15 g 1-Nitronaphthalin und 30 g Zeolith H-Y der Firma Degussa (zu Pulver zermahlenes Extrudat, SiO₂/Al₂O₃ = 23) wurden vorgelegt und auf 80°C erwärmt. Dann wurden 44 g 99 gew.-%ige Salpetersäure innerhalb von 30 Minuten zugegeben. Anschließend wurde der Ansatz 2 Stunden bei 80°C gerührt. Die Aufarbeitung erfolgte durch Zugabe von vollentsalztem Wasser und anschließende Extraktion mit Dichlormethan.
Ergebnisse:
- Umsatz an 1-Nitronaphthalin: 25,6 %
- Regioselektivität zum 1,5-Dinitronaphthalin: 55,5 %
- Regioselektivität zum 1,8-Dinitronaphthalin: 37,3 %
- Molverhältnis 1,5-/1,8-Dinitronaphthalin: 1,49
- Ausbeute an -1,5-Dinitronaphthalin: 14,2 %

### Beispiel 23

### Nitrierung von 1-Nitronaphthalin mit Salpetersäure in Gegenwart von Sulfolan und Zeolith H-Y

302 g Sulfolan, 15 g 1-Nitronaphthalin und 30 g Zeolith H-Y der Firma PQ (Pulver, SiO₂/Al₂O₃ = 60) wurden vorgelegt und auf 80°C erwärmt. Dann wurden 52 g 99 gew.-%ige Salpetersäure innerhalb von 30 Minuten zugegeben. Anschließend wurde der Ansatz 2 Stunden bei 80°C gerührt. Die Aufarbeitung erfolgte durch Zugabe von vollentsalztem Wasser und anschließende Extraktion mit Dichlormethan.
Ergebnisse:
- Umsatz an 1-Nitronaphthalin: 53,3 %
- Regioselektivität zum 1,5-Dinitronaphthalin: 51,7 %
- Regioselektivität zum 1,8-Dinitronaphthalin: 41,3 %
- Molverhältnis 1,5-/1,8-Dinitronaphthalin: 1,25
- Ausbeute an 1,5-Dinitronaphthalin: 27,6 %

### Beispiel 24 (Vergleichsbeispiel)

### Nitrierung von 1-Nitronaphthalin mit Salpetersäure in Gegenwart von Sulfolan

302 g Sulfolan und 25 g 1-Nitronaphthalin wurden vorgelegt und auf 80°C erwärmt. Dann wurden 55 g 99 gew.-%ige Salpetersäure innerhalb von 40 Minuten zugegeben. Anschließend wurde der Ansatz 2 Stunden bei 80°C gerührt. Die Aufarbeitung erfolgte durch Zugabe von vollentsalztem Wasser und anschließende Extraktion mit Dichlormethan.
Ergebnisse:
- Umsatz an 1-Nitronaphthalin: 16,3 %
- Regioselektivität zum 1,5-Dinitronaphthalin: 36,0 %
- Regioselektivität zum 1,8-Dinitronaphthalin: 53,5 %
- Molverhältnis 1,5-/1,8-Dinitronaphthalin: 0,67
- Ausbeute an 1,5-Dinitronaphthalin: 5,8 %

### Beispiel 25

### Nitrierung von 1-Nitronaphthalin mit Salpetersäure in Gegenwart von Nitromethan und Zeolith H-Y

300 g Nitromethan, 18 g 1-Nitronaphthalin und 12 g Zeolith H-Y der Firma PQ (Pulver, SiO₂/Al₂O₃ = 30) wurden vorgelegt und auf 80°C erwärmt. Dann wurden 26g 99 gew.-%ige Salpetersäure innerhalb von 60 Minuten zugegeben. Anschließend wurde der Ansatz 210 Minuten bei 80°C gerührt. Nach dieser Zeit wurde das Reaktionsgemisch vom Zeolith abgetrennt und dieser noch zweimal mit je 500 mL 1,2-Dichlorethan und zweimal mit je 500 mL Dichlormethan extrahiert (je 30 Minuten bei Raumtemperatur). Die Produktphasen wurden aufgearbeitet und die Zusammensetzung des dabei erhaltenen festen Rohproduktes gaschromatographisch bestimmt, wobei die Quantifizierung rnit Hilfe eines inneren Standards (n-Hexadecan) erfolgte:
- Isomerenanteil des 1,5-Dinitronaphthalins: 50,2 %
- Isomerenanteil des 1,8-Dinitronaphthalins: 42,6 %
- Verhältnis 1,5-/1,8-Dinitronaphthalin: 1,18

Das Rohprodukt war ein Dinitronaphthalin-Isomerengemisch, welches kein 1-Nitronaphthalin, keine Trinitronaphthaline und keine sonstigen Nebenprodukte enthielt. Es wurden 19,2 g dieses Rohproduktes enthalten. Dies entspricht einer Ausbeute an 1,5-Dinitronaphthalin von 42,5 %.

## Patentansprüche

1. Verfahren zur Herstellung eines Dinitronaphthalin-Isomerengemisches, bei dem man Naphthalin und/oder 1-Nitronaphthalin mit Salpetersäure in Gegenwart von Zeolithen der struktur- typen MFI, MOR, BEA, FAU, MEL, EMT, MTW, LTL, MWW, RHO, FER oder HEU in der sauren H⁺-Form umsetzt.

2. Verfahren nach Anspruch 1, bei dem die Umsetzung in Gegenwart mindestens eines organischen Lösemittels durchgeführt wird.

3. Verfahren nach Anspruch 2, bei dem als organische Lösemittel n-Hexan, n-Heptan, Cyclohexan, Ligroin, Dichlormethan, Chloroform, 1,2-Dichlorethan, 1,2-Dichlorpropan, Essigsäure, Nitromethan, Nitroethan oder Sulfolan eingesetzt werden.

4. Verfahren nach einem der Ansprüche 1 bis 3, bei dem die Konzentration der eingesetzten Salpetersäure zwischen 40 und 100 Gew.-% beträgt.

5. Verfahren nach einem der Ansprüche 1 bis 4, bei dem pro Mol Naphthalin 0,2 bis 40 Mol Salpetersäure und/oder pro Mol 1-Nitronaphthalin 0,1 bis 20 Mol Salpetersäure eingesetzt wird.

6. Verfahren nach einem der Ansprüche 1 bis 5, bei dem die Salpetersäure in einer Mischung mit Schwefelsäure und/oder Phosphorsäure eingesetzt wird.

7. Verfahren nach einem der Ansprüche 1 bis 6, bei dem Zeolithe vom Typ H-Y, H-beta, H-Mordenit oder H-ZSM-5 verwendet werden.

8. Verfahren zur Herstellung von 1,5-Diaminonaphthalin, bei dem man ein Dinitronaphthalin Isomerengemisch nach einem Verfahren nach Anspruch 1 herstellt und dieses durch fraktionierte Kristallisation in die isomeren Dinitronaphthaline trennt und das so erhaltene 1,5-Dinitronaphthalin zum 1,5-Diaminonaphthalin hydriert.

9. Verfahren zur Herstellung von 1,5-Diisocyahatonaphthalin, bei dem man 1,5-Diamiaonaphthalin nach einem Verfahren nach Anspruch 8 herstellt, und anschließend das entstandene 1,5-Diaminonaphthalin zum 1,5-Diisocyanatonaphthalin phosgeniert.

## Claims

1. Process for the production of a dinitronaphthalene isomer mixture, by reacting naphthalene and/or 1-nitronaphthalene with nitric acid in the presence of zeolites of the structure types MFI, MOR, BEA, FAU, MEL, EMT, MTW, LTL, MWW, RHO, FER or HEU in the acidic H⁺ form.

2. Process according to Claim 1, in which the reaction is performed in the presence of at least one organic solvent.

3. Process according to Claim 2, in which use is made as organic solvents of n-hexane, n-heptane, cyclohexane, ligroin, dichloromethane, chloroform, 1,2-dichloroethane, 1,2-dichloropropane, acetic acid, nitromethane, nitroethane or sulfolane.

4. Process according to any of Claims 1 to 3, in which the concentration of the nitric acid used is between 40% and 100% by weight.

5. Process according to any of Claims 1 to 4, in which 0.2 to 40 mol of nitric acid per mole of naphthalene and/or 0.1 to 20 mol of nitric acid per mole of 1-nitronaphthalene are used.

6. Process according to any of Claims 1 to 5, in which the nitric acid is used in a mixture with sulphuric acid and/or phosphoric acid.

7. Process according to any of Claims 1 to 6, in which zeolites of the H-Y, H-beta, H-mordenite or H-ZSM-5 type are used.

8. Process for the production of 1,5-diaminonaphthalene, by preparing a dinitronaphthalene isomer mixture by a process according to Claim 1 and separating this mixture by fractional crystallization into the isomeric dinitronaphthalenes and hydrogenating the resultant 1,5-dinitronaphthalene to form 1,5-diaminonaphthalene.

9. Process for the production of 1,5-diisocyanatonaphthalene, in which 1,5-diaminonaphthalene is prepared by a process according to Claim 8 and subsequently the 1,5-diaminonaphthalene formed is phosgenated to form 1,5-diisocyanatonaphthalene.

## Revendications

1. Procédé de production d'un mélange d'isomères de dinitronaphtalène, dans lequel on convertit du naphtalène et/ou du 1-nitronaphtalène avec de l'acide nitrique en présence de zéolithes des types de structure MFI, MOR, BEA, FAU, MEL, EMT, MTW, LTL, MWW, RHO, FER ou HEU sous la forme acide H⁺.

2. Procédé selon la revendication 1, dans lequel on effectue la conversion en présence d'au moins un solvant organique.

3. Procédé selon la revendication 2, dans lequel on utilise comme solvant organique du n-hexane, n-heptane, cyclohexane, ligroine, dichlorométhane, chloroforme, 1,2-dichloroéthane, 1,2-dichloropropane, acide acétique, nitrométhane, nitroéthane ou sulfolane.

4. Procédé selon l'une quelconque des revendications 1 à 3, dans lequel la concentration de l'acide nitrique utilisé est comprise entre 40 et 100 % en poids.

5. Procédé selon l'une quelconque des revendications 1 à 4, dans lequel on utilise par mole de naphtalène 0,2 à 40 moles d'acide nitrique et/ou par mole de 1-nitronaphtalène 0,1 à 20 moles d'acide nitrique.

6. Procédé selon l'une quelconque des revendications 1 à 5, dans lequel on utilise l'acide nitrique en mélange avec de l'acide sulfurique et/ou de l'acide phosphorique.

7. Procédé selon l'une quelconque des revendications 1 à 6, dans lequel on utilise des zéolithes du type H-Y, H-β, H-mordénite ou H-ZSM-5.

8. Procédé de production de 1,5-diaminonaphtalène, dans lequel on produit un mélange d'isomères de dinitronaphtalène par un procédé selon la revendication 1 et on sépare ce dernier par cristallisation fractionnée en ses dinitronaphtalènes isomères et on hydrate le 1,5-dinitronaphtalène ainsi obtenu en 1,5-diaminonaphtalène.

9. Procédé de production de 1,5-diisocyanatonaphtalène, dans lequel on produit du 1,5-diaminonaphtalène par un procédé selon la revendication 8 et on phosgène ensuite le 1,5-diaminonaphtalène obtenu en 1,5-diisocyanatonaphtalène.
